# EUROPEAN PATENT APPLICATION

(11) **EP 1 213 347 A1**
(43) Date of publication of application: **12.06.2002**
(21) Application number: 00204181.2
(22) Date of filing: 24.11.2000
(51) Int. Cl.: C12N 1/04

(54) **A method for preserving cells by processing the same into dry products**

(71) Applicant: TDI Tournois Dynamic Innovations B.V., 6708 PW Wageningen (NL)
(72) Inventor: Jongboom-Yilmaz, Gulden, 6671 AE Zetten (NL); Jongboom, Remigius Oene Jules, 6671 AE Zetten (NL); Turnois, Huibert, 3911 JC Rhenen (NL)
(74) Representative: Jorritsma, Ruurd

(57) **Abstract**

The invention relates to a method for formulating drying, preserving, immobilising and/or encapsulating cells with minimal loss of viability. In the method cells are mixed with a matrix material which is capable of absorbing water and, preferably, the mixture is subjected to a treatment which effects the formation of a continuous phase of the matrix encapsulating the cells. Such treatment preferably is extrusion.

## Description

The present invention relates to a method for drying, preserving, immobilising and/or encapsulating of living cells with minimal loss of vitality.

The invention is *inter alia* based on the surprising discovery that cells, while in mixture with and/or while being mixed with certain matrix materials as further described below, may be subjected to relatively harsh treatments like extrusion, oven drying, drum drying of cells, even at the conditions further specified below, while retaining cell vitality, so as to provide advantageous mixtures of the cells and the matrix material(s).

For instance, the mixtures thus obtained may provide ease of storage, transportation and handling, and may also be stored for prolonged periods of time without significant reduction of quality and/or significant loss of cell viability.

Also, such mixtures may be provided for a range of intended final uses and/or applications, also as further described below.

Although the invention is not limited to any specific mechanism or explanation, it is assumed that the method of the invention is probably based at least in part, on rapid redistribution of water over cells and the suspension medium towards the matrix after which the relatively dry cells are physically protected due to low water content (low water activity), and fixation by the matrix.

A hydrophilic oligomer or polymer (proteins, carbohydrates, hydrocolloids etc.), osmotically active components or mixtures can be used as matrix materials. In order to improve the working action of the matrix or adapt the physical properties for application other additives such as plasticizers, emulsifiers, salts, cryoprotectants or hydrophobic components can be incorporated.

It has been found that processing as described below makes cells more resistant to temperature, shear and pressure effects and allows further processing of cells in various ways while maintenance cell integrity and cell function. The cells and the polymer-oligomer/additive mixture in this pre-dried state, may be further processed in order to effect a continuous phase of the matrix in which the cells are encapsulated and/or may be further processed in order to be dried, coated or shaped, by means of moulding techniques, thermoplastic processing such as extrusion, kneading or conventional/non conventional drying such as fluidised bed dryer, vacuum oven, drum drier, coating equipment and the like.
Accordingly, the present invention relates to a method, which is assumed to be based on a concept of a redistribution- of water between cells and/or parts of cells (and viruses, proteins, vesicles, micelles, spores etc), which can be followed by further processing e.g. further drying, shaping, encapsulating, or coating with the intention to make the cells less vulnerable for temperature, shear or pressure effects and obtaining a formulation which can be used for controlled release applictions. This method can be applied to prepare products involving cells, eukaryotic and/or prokaryotic, for protection, immobilisation or encapsulation purposes.

Other aspects, embodiments and advantages of the invention will become clear from the further description given hereinbelow.

In a first aspect, the present invention relates to a method for formulating, treating and/or processing cells, in particular for use in drying, preserving, immobilising and/or encapsulating of cells, said method at least comprising the step of:
(a) mixing the cells with at least one matrix material, in which the ratio of the cells (cell suspension) and the at least one matrix material is between 0.001% and 80 % and in particular between 10% and 50%,
   and which method may further comprise the step of:
(b) subjecting the one matrix material, before, during and/or after mixing with the cells in step (a), to at least one treatment involving an input of thermal and/or mechanical energy such as shear and/or pressure, in order to effect the formation of a continuous phase of the matrix in which the cells are or will be encapsulated, or any combination thereof. A preferred example of such a treatment is extrusion.

In addition, the method of the invention may also comprise at least one further step of:
(c) subjecting the mixture obtained after step (a), or the mixture obtained after steps (a) and (b), to at least one treatment in which:
   - said mixture is (further) dried, e.g. at a temperature of between 50 °C and 200°C, for a time of between 3 min and 3hrs, or at room temperature between 20°C and 35°C for between 3 hrs and 3 days.
   - said mixture is formed, e.g. into pellets, granules, spheres, powder, flakes or tablets;
   - said mixture is coated;
   - said mixture is packaged;
   - said mixture is stored and/or transported prior to final use;
   - said mixture is dispersed or suspended
or any combination thereof.

The invention also relates to the mixtures of cells and at least one matrix material thus obtained.

In the above method, the cells added to step (a) - and/or used in step (a) in the form of a powder or a paste but preferably in a form of a suspension of the cells in a liquid medium, and in particular in the form of a suspension of the cells in an aqueous medium such as water or a buffer solution.
Preferably, such a suspension comprises between 10² and 10¹³ cells per ml. of liquid/aqueous medium, in particular between 10⁷ and 10¹⁰ cells per ml. of liquid/aqueous medium.

For instance, when the cells are in the form of a cell suspension as mentioned above, the matrix material may be added in an amount of between 5 % and 95% of the total composition in particular between 40 % and 60% of the total composition.

However, it should be noted that in its broadest sense, the invention is not limited to the specific form and/or to the specific manner in which the cells and the matrix material are added to each other and/or mixed with each other, as long as a suitable mixture of the cells and the matrix material(s) is obtained, wherein preferably the cells are encapsulated in a continuous phase of the matrix.

The above steps (a) and/or (b) may be carried out in suitable manner known per se, e.g. as further described below. For instance, step (a) may be carried out by mixing the cells and the matrix material in a suitable mixing apparatus, such as a ribbon blender or a varimixer. In one particularly preferred embodiment, step (a) is carried out in an extruder.

Step (b) may for instance be carried out by extrusion, compression moulding, (high) shear mixing, drum drying, oven drying, static mixer e.g. so as to subject (the mixture containing) the cells to at least one of the conditions of temperature, shear forces and/or pressure, as mentioned above, preferably to effect the formation of a continuous phase of the matrix.

Also, the above steps (a) and (b) may be carried out as separate steps or may be combined into a single step. When the above steps (a) and (b) are combined, they are preferably carried out by extrusion as further described below.

When the above step(s) (a) and/or (b) are carried out by extrusion, any suitable extruder known per se may be used, for instance a co-rotating twin screw extruder, which may be operated under suitable conditions, e.g. suitable temperature, pressure, shear and suitable retention time.

The matrix material used is preferably a polymer, oligomer or a mixture of two or more polymers or oligomers, for instance chosen from one or more biopolymers including but not limited to proteins, carbohydrates (or derivatives thereof); and/or one or more synthetic polymers; or any combination of such synthetic polymers and/or biopolymers.

Preferably, the matrix material comprises at least one hydrophilic polymer or a mixture of two or more hydrophilic polymers or oligomers and/ or components The water absorbing capacity of such materials can be between 0.2 g water/g material to 1000 g water/g materialPreferably, the water absorbing capacity of such materials is at least 1 g water/g material, more preferably at least 5, 25, or 100 g water/g material, and most preferably at least 500 g water/g material.

For instance, the one or more polymer(s) that make up the matrix material can be chosen from starch (native or modified), (cyclo-) dextrins, inulin, gelatine, casein, proteins from sources like wheat, soy, corn or other grains, hydrocolloids such as carragenans, alginates, xantan gum, gum arabic, guar flour, guar gum, agar, tragacanth, karaya, locust bean gum, fibres, modified celluloses, pectin, cellulose esters, cellulose ethers; or any combination thereof.

The use of starch and starch derivatives (such as modified starches including but not limited to pre-gelatinised and/or destructrized starches) is particularly preferred.

The cells may be any desired cell, mixture of cells or part(s) of cells. The cells may be prokaryotic cells, including but not limited Gram-positive, Gram-negative or Archeabacterial species, or the cells may by eukaryotic cells, including but not limited to yeast cells, fungal cells, cells of algae, animal cells and/or plant cells. Although scientifically not correct, the term cells as used herein, should be understood to also include viruses, including but not limited to virus particles, viroids, and (bacterio) phages. For this purpose a viable virus or part thereof, is understood to mean a virus capable of replication in a suitable host cell or host organism. Parts of cells may be membranes, organelles such as mitochondria, microbodies (peroxisomes), microsomes or other parts from the endoplasmic reticulum or Golgi apparatus, or proteins or nucleic acids.

In one particularly advantageous aspect of the invention, the cells are living cells and/or viable cells. In this aspect, the invention may be used to provide a mixture of said cells with the at least one matrix material, in which the cells are still viable, e.g. to a degree sufficient for the intended final use.

Accordingly, in this aspect of the invention, (the conditions for carrying out) step (a), step (b) and optionally step (c) are chosen such that during said steps, the viability of the cells does not decrease by more than 60 %, preferably by no more than 20 %, and in particular by no more than 10%; in which the viability of the cells is calculated as: [the total number of viable cells added to, or present in, the mixture] divided by [the total number of cells (originally) added to, or present in, the mixture], multiplied by [100%].

In the above method, also one or more additional components may be added to the mixture of the cells and the at least one matrix material, e.g. during step (a) and/or during step (b) (and optionally also during step (c)). Such additives may for instance be added to the cells together with, as part of, and/or in admixture with the at least one matrix material.

For instance, such additives may be chosen from
- one or more osmotically active agents, such as carbohydrates, proteins, modified carbohydrates, synthetic hydrophilic polymers, sugars, mono- and di-saccharides, alcohols, salts, glycerol, and the like;
- one or more cryoprotectants, such as carbohydrates, modified carbohydrates, sugars, mono and di-saccharides, alcohols, amides, glycerol, propylene glycol, DMSO, PEG 200-600, sucrose, glucose, ertrihol, xylitol, inositol raffinose, trehalose, salts, and the like;
- one or more hydrophobic components, lubricants, such as synthetic polymers, emulsifiers, fatty acids, fats, waxes , or mixtures and the like;
- one or more emulsifiers, such as cationic, anionic or non-ionic surface active agents from plant or animal origin such as polyoxyethylene sorbitan esters, sucrose esters of fatty acids, propylene glycol esters, polyglycerol esters of fatty acids and lecithins;
- plasticizers, such as water, glycerol, sugars, sugar solutions, PEGs and the like;
- fillers, such as ceramic materials, biological fillers, fibres, and the like;
or any combination thereof.

Also, the incorporation into the mixture of one or more (further) carriers, adjuvantia or excipientia (i.e. in addition to the matrix materials), although usually not required, is not excluded from the scope of the invention.

In another aspect, the invention also relates to a mixture of cells and at least one matrix material, and optionally one or more additives, obtainable and/or obtained via the method described above.

Thus in one aspect the invention relates to a mixture having e.g. the general composition:
(1) cell suspension: between 10 % and 50% wt.%;
(2) matrix material(s) between 50 %and 90 wt.%;
(3) further additives between 1% and 30 wt.%;
to a total of 100 wt.% of the cells (1), the matrix materials (2) and the further additives (3).
or any combination thereof. It is to be understood that the percentages of the various components in the mixture relate to the amounts of the various components as added to or incorporated into the mixture. These percentages may be different in the final mixture as obtained after processing as such processing may involve drying and/or adding water and/or other volatile compounds. Preferably, the mixture is further characterized in that the matrix materials are in a continuous phase encapsulating the cells, whereby at least 50%, preferably at least 60%, more preferably at least 80% and most preferably at least 90% of the cells are viable.

The mixtures and products obtained according to the invention have a number of advantages, including but not limited to:
- the mixtures of the invention provide for ease of handling, storage, transportation and use, e.g. compared to suspensions of cells;
- the mixtures of the invention may be stored for prolonged periods of time without substantial reduction of quality and/or - when said mixtures comprise living cells - without substantial reduction of cell viability. For instance, the mixtures,products of the invention may be stored under conventional storage conditions or at higher storage temperatures from 4 to 30 °C for a time of at least 1 month, and up to 12 months or more. During such storage time, the viability of the cells - calculated essentially as mentioned above, starting from the cell viability of the mixtures as obtained using the method of the invention - is preferably is not reduced by more than 60 % and preferably by no more than 20%
- the cells in the encapsulated form can be used as starter cultures providing the ease for growth and decreasing the pre adapting period that the cells are under shock conditions
- the formulation of the mixtures and products can be adjusted to give several release patterns such as triggered release.
- the formulation of the mixtures and products can be used targetted delivery of cells for several purposes such as vaccination or applications for functional foods.
- the formulation of the mixtures and products can be adjusted to provide gastric resistence.
- the formulation provides excellent protection against oxygen for example for in case of encapsulation of anaerobic bacteria.
The mixtures and products of the invention provide protection against contamination from external environment or of the external environment.or any combination thereof. The mixtures of the invention may be provided and/or used for any purpose or application known per se. Usually, the intended final use or application will also to a large extent determine the specific choice of the cells, the matrix materials and the optional additives which are incorporated into the mixtures of the invention, and may also determine to a large extent the specific manner and conditions under which the above steps (a) to (c) are carried out. All this will be within the skill of the artisan, based upon the disclosure herein.

Some particularly advantageous applications of the invention include, but are not limited to:
- providing mixtures of matrix materials and at least one suitable lactic acid bacterium, e.g. for use as a starter culture in the preparation of dairy products;
- shaped articles with embedded cells for tissue engineering purposes;
- encapsulation of e.g. *Bifidobacterium* for colon targetting;
- encapsulation of vaccines to be applied for oral or mucosal vaccination for animal or human use;
- encapsulation of tissue cells which secrete hormones for *in vitro* secretion of hormones or this formulation can be implanted in a diabetic mammal;
- encapsulation of probiotics for taste masking purposes;
- immobilisation of cells for production of several metabolites or to be used in fermentation technologies;
- As a way to increase cell concentration;
- for waste treatment application of soil and water;
- as formulations for soil quality improvement;
- as fertilisers;
- as formulations for pest control;
- for diagnostics;
- encapsulation for protection against patogenes;

Prior to the present invention, it was generally assumed that high temperatures during drying processes are the main reason for loss if cell vitality. Technologies for drying of living cells try to lower temperature during drying or to reduce the time during which high temperatures are applied. However, it is also known that drying of membranes according to these known techniques leads to inherent and irreversible changes in the cell membranes due to e.g. changes in protein conformations. In membranes lower water concentrations may lead to phase separation of membrane lipids and a redistribution of membrane proteins e.g. due to changes in the phase behaviour of membrane lipids. Also lower water content may lead to formation of non-bilayer structures in the membranes. Proteins may change their conformation or associations. These inherent changes can cause a permanent loss of protein function, membrane integrity, loss of valuable cell content and thereby cell inactivity or death.

The relative contribution of this type of disruption is probably more important when the drying process is slower and the temperatures allow formation of such structures. It is important to choose the temperature and other conditions to prevent these changes from happening. One possibility to achieve this is pre-drying at lower temperatures, lowering the water content at temperatures that do not allow (thermal) disruption of membranes and enzymes, followed by a short higher temperature drying process. In the second phase proteins and membranes are more resistant against higher temperatures due to the lower water activity (hydrolysis, denaturation, changes in membrane structure need sufficient water). This is a process with highly complex kinetics of various separate reactions.

According to the invention, it was now found that a pre-treatment for the replacement/redistribution of water from the cells to a matrix material as described herein - e.g. when carried out under the conditions as described in the examples - results in a system in which the cells are less vulnerable for further processing.

In the redistribution step, total absorption of the water in the suspension medium and at partial absorption of the water content of the cell is achieved by a hygroscopic matrix and/or other osmotically active substances. In this way the water activity is lowered by redistribution of the water from the suspension medium and the cells towards the matrix. In a later step, the temperature can become higher and the water from the matrix can be dried, without substantially damaging the cells.

During rehydration the temperatures are lower and the cell membrane is the first structure to rehydrate.
The matrix also provides an excellent protection in a number of ways: it maintains the cell shape, prevents membranes form fusing, loss of content etc. and acts as a barrier against oxygen uptake and thereby oxidation. At the same time it provides a matrix in which also cryoprotectants, osmotically active compounds can be added to further stabilise the membranes. Examples of such cryoprotective and/or osmotically active compounds include, but are not limited to such as carbohydrates, modified carbohydrates, sugars, mono- and di-saccharides, alcohols, amides, glycerol, propylene glycol, DMSO, PEG 200-600, sucrose, glucose, ertrihol, xylitol, inositol raffinose, trehalose, salts, and the like. Addition of osmotically active compounds will lead to improved drying properties of the matrix.

Accordingly, the formulation comprises a matrix material or combination of matrix materials, cell culture, and additives such as osmotically active agents, cryoprotectants, emulsifiers or hydrophobic components.

The redistribution or replacement of water can be achieved simply by admixing the cells, the matrix and additives if required prior to further processing using a mixing device. In this stage preferably unbound water is absorbed by the matrix and/ or the additives, osmotically active agents and the water activity is substantially lowered.

The step of redistribution or replacement of water can be followed by further processes for drying and/or shaping purposes using conventional or non-conventional drying equipment such as ovens, vacuum ovens, deep bed dryers, fluidised bed dryers, belt dryers, spray dryers, spray chilling, Wurster dryer, drum dryer, centrifugal extrusion and the like. Sufficient drying such as towards moisture contents, down to 5 - 15% of moisture can be achieved, which assures prolonged storage stability. In embodiments of the invention, products can be shaped using thermoplastic processing such kneading, extrusion and moulding techniques such as compression moulding and injection moulding and pressed into pellets or tablets, cut or ground. The obtained products may than be, coated/surface treated using (film forming) substances for protection in aqueous or alcoholic solutions or lipophilic, conditions such as, carbohydrates, proteins, modified carbohydrates or proteins, shellac, zein, chitosan, chitin, emulsifiers, fatty acids, fat, waxes, synthetic polymers, or mixtures and the like. In embodiments where coating is applied, conventional spray nozzles, conventionally known fluid bed coating apparatus or other coating apparatus and methods such as coating pans or coating drums can be used.

All steps of processing may just as well be combined in one continuous processing step such as extrusion. Extrusion process can be performed using single or double screw, co-rotating, counter-rotating or multi-screw extruders. Processing temperatures can vary between 30 to 150°C and are preferably between 30 to 120 °C. Redistribution of water in the system can be achieved in the premixing stage or in the beginning of the extruder, feeding in a suspension of cells just after the matrix material feed. In the following zones, evaporation of the excess water or at least a part of it, can be optionally achieved by means of increased temperatures up to 150 and preferably 120°C, application of a vent (or series) or a vacuum pump (or series) down to levels sufficient enough to shape the obtained mixture. The moisture reduction within the extruder also provides for the attainment of a formable composition capable of being formed into discrete, substantially uniform pieces.

Additives and/or other ingredients can be admixed with the matrix material prior to extrusion or can just as well be added during extrusion, together with the suspension or downstream in the extruder.
In the embodiments of the present invention at least one osmotically active ingredient or component or a matrix (mixture) is desired. A hydrophilic polymer can be used as the matrix. This polymer may be a biopolymer such a carbohydrate or a protein, such as starch (native or modified), (cyclo-) dextrins, inulin, gelatine, casein, proteins from sources like wheat, soy, corn or other grains, hydrocolloids such as carragenans, alginates, xantan gum, gum arabic, guar flour, guar gum, agar, tragacanth, karaya, locust bean gum, fibres, modified celluloses, pectin and the like or other hydrophilic polymers such as cellulose esters, cellulose ethers and the like or combinations of these. A synthetic, water soluble or swellable polymer or oligomer can be applied as a matrix in a similar way as described above as well. The optimal composition can for example be achieved using a mixture of destructrized and/ or low molecular weight starches and granular starch. The amount of granular starch can be between 0 to 90 % of the total composition and most preferably 10-40% of the total composition. Substances that have a water binding capacity can be incorporated additionally, such as sugars, mono- or di-saccharides, starch hydrolyzate products such as dextrins or any other hygroscopic substance such as salts or other inorganic compounds (molsieve etc.). Furthermore, other ingredients can be incorporated; such as plasticizers, cryoprotectants, degrading enzymes, emulsifiers and hydrophobic additives such as oils, fats waxes or hydrophobic polymers in any stage of processing for further protection, stabilisation, for modified dissolution or rehydration behaviour, controlled or delayed release of the included cells. Matrix components or additives can be used to increase/decrease the rate of dissolution, rehydration or the release. The products can be readily dissolving or dispersible or suspendable.

The products can be produced in many forms or shapes e.g. in a powder form, as granules, flakes, particles, pellets, tablets and capsules or even as continuous objects like films, tubes or rods.

The moisture content of the final product may be from 2% to 25% and preferably from 10-12% on the basis of total composition by weight.

The method of the present invention can be used to prepare products to be used for direct consumption, incorporated into foods (functional foods), as starter cultures for food, chemical industry or fermentation technologies, for better storage stability, or resistance and in pharmaceutical industry for oral targeting, vaccination, tissue engineering, implantation, gene delivery and thereof.

The present invention is further illustrated by the following, non-limiting examples where all parts, percentages, proportions, and ratios are by weight and all temperatures are in °C unless otherwise indicated;

The analysis of the examples for the survival rate was performed by extracting the cells from the matrix by degrading the matrix for 2 to 5 hours and by using a stomacher for disrupting the particles for a better performance in extraction. The extracted solution was plated to get a reading for the number of cells/ml suspension and thus calculating the survival ratio by comparing this number with the number of unprocessed suspension of cells.

The following examples serve to illustrate the invention and by no means should be construed so as to limit the scope of the invention.

### Examples

### Example 1

A suspension of *Lactococcus lactis* (9.70E +08 cfu/ml) was admixed (blended) with destructrized potato starch. The premix was obtained by mixing these ingredients at room temperature, for 10 minutes using a spiral mixing device coupled with the varimixer.

The amount of suspension was 20 % based in total composition. It was seen that this resulted in a sticky mixture, which was due to the high water uptake of the starch component upon mixing.

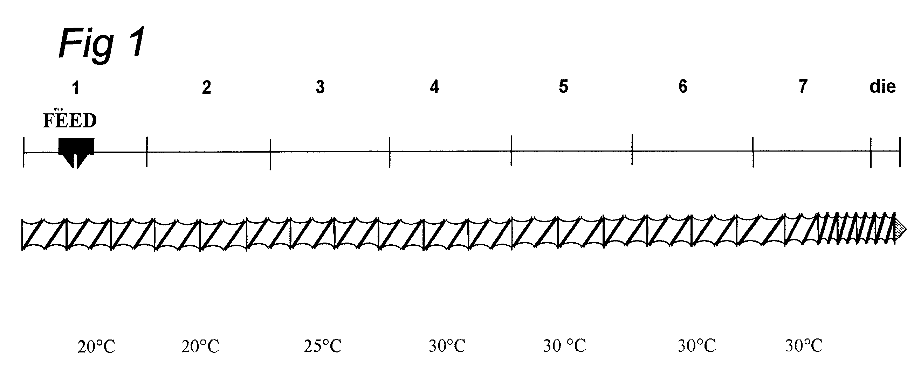

This mixture/blend, was fed into the first barrel of a twin screw co-rotating extruder, at a rate of 2.5 kg/h. The extruder used was a Werner and Pfleider ZSK 25 with a screw diameter of 25 mm and a length of 28 D. The screw speed employed was 100 rpm. Screw configuration was composed of only positive pitch elements. The screw configuration and barrel temperature profile can be seen in figure 1. The measured torque value was at 17% while the die pressure remained at 8 bars during processing. Melt temperature of the mixture was 33 °C as measured at the die. It was observed that a sufficient mixing and forming was achieved by the applied processing conditions.

It was seen that there was no loss of living cells in the first admixing stage (redistribution of water) and there was a survival ratio of 30 % in counted number of cells after the extrusion process.

### Example 2

In this example, native potato starch was added in the first blending stage. The ratio of the added native potato starch was 40% on total composition, which was composed of 40% of destructrized starch and 20 % suspension of *Lactococcus lactis*.

The extrusion was performed in the same way as example 1. The torque value measured was 17 %, melt temperature 38 °C and the die-pressure 17 bars.

The survival ratio after extrusion was 27 % when compared with the count in the beginning suspension (original count).

### Example 3

In this example, effect of adding low molecular weight carbohydrates was evaluated. Sugar was added in the premixing stage in a ratio of 10% on total composition.

The same extrusion compositions were used and the measured values for torque, melt temperature and die pressure were 13%, 33 °C and 6 bars respectively.

The survival ratio after processing was found to be 60 % of the original count where as the survival ratio after 7 days of storage at 4 °C was found to be 55% of the original count.

### Example 4

This example was a comparative example to the example 3 except that the extrusion was performed at a screw speed of 350 rpm instead of 100. The measured values of the torque, melt temperature and die pressure were 18, 38 °C and 17 bars respectively.
The survival ratio after processing was 3% of the original count.

### Example 5

In this example the addition of glycerol was investigated. Glycerol was added in the premixing stage in an amount of 10 % on total composition.

Extrusion conditions were kept the same as in example 1. The measured values of torque melt temperature and die pressure were 14, 32 °C and 10 bars respectively.

The survival ratio after processing was found to be 87 % of the original count where as the survival ratio after 7 days of storage at 4 °C remained unchanged. When the sample of this example was stored at 4 °C for 35 days the survival ratio was found to be 30% of the original count.

### Example 6

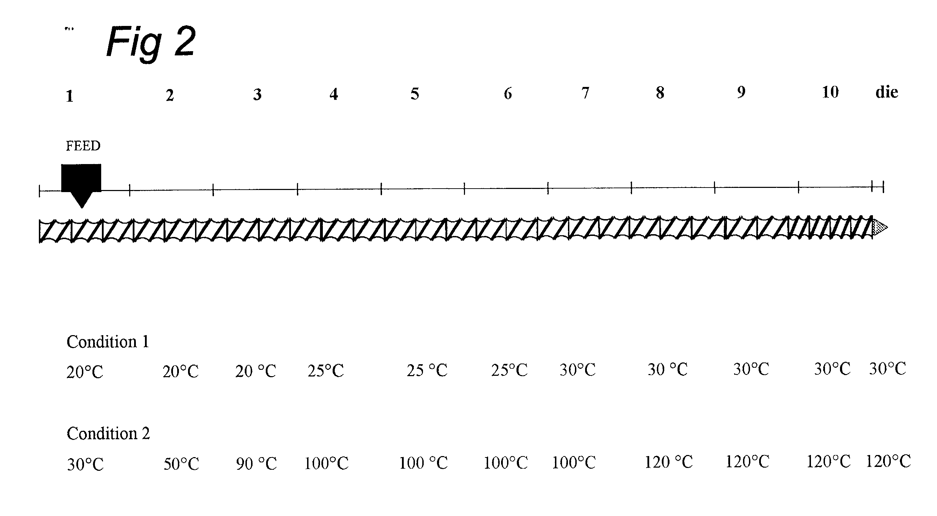

In this example the same composition and extrusion conditions were repeated in a twin screw, co-rotating self wiping extruder (Berstorff, D=25 mm, L=40D). The screw configuration and the set temperatures of the barrel for the process can be seen in fig. 2 (condition 1). The measured values of torque melt temperature and die pressure were 17, 35 °C and 9 bars respectively.

The micro-organism was a baker's yeast (*Saccharomyces cerevisiae*, 1.16875E +09 cfu/ml). The survival ratio after processing was found to be 93 % of the original count.

### Example 7

In this example the same composition and extrusion conditions were repeated as in example 6 except the micro-organism was *Bflidobacterium bifidum* (1.23E+09 cfu/ml). The measured values of torque melt temperature and die pressure were 15, 36 °C and 8 bars respectively. The survival ratio after processing was found to be 97 % of the original count.

### Example 8

In this example the same composition was maintained as in example 6 and the micro-organism used was baker's yeast (1.16875E +09 cfu/ml). The set temperatures for the extrusion was as given in condition 2, using the previously used screw configuration. The screw speed was 100 rpm. The measured values of torque melt temperature and die pressure were 12, 124 °C and 14 bars respectively The survival ratio after processing was found to be 22 % of the original count.

### Example 9

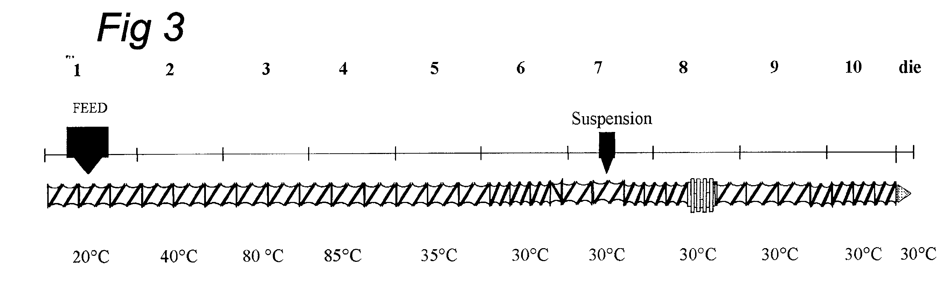

In this example a premix was of native potato starch, destructrized potato starch and glycerol was fed to the extruder. The suspension of baker's yeast (1.16875E +09 cfu/ml) was introduced downstream in the extruder at the 7 th. zone using a piston pump (Pro Minet Verder BV, Germany). The screw speed employed during processing was 100 rpm. The set temperatures and the screw configuration are demonstrated in fig. 3. The measured values of torque melt temperature and die pressure were 22, 41 °C and 21 bars respectively The survival ratio after processing was found to be 46 % of the original count.

### Example 10

In this example a set of samples were exposed to different temperatures for different period of time. Samples were prepared making a premix of the suspension with the micro-organism (20% on total composition), destructrised potato starch, native potato starch and glycerol. 50 grams of the premix was weighed and placed in aluminium trays to employ the process. The timing for the the was started when the dry sample temperature reaches the set temperature of the oven.

Sample compositions, experimental conditions and survival ratios can be seen in Table 1.

**Table 1.**

| Drying experiments | | | |
|---|---|---|---|
| **Type of M.O.** | **Oven T (°C)** | **Exposure t (min)** | **Survival ratio (%)** |
| *Bifidobacterium* | 120 | 10 | 97.2 |
| *Bifidobacterium* | 120 | 15 | 95.3 |
| *Bifidobacterium* | 70 | 30 | 93.5 |
| *Bifidobacterium* (control) | 70 | 30 | 88.5 |
| *Saccharomyces* | 120 | 15 | 70.6 |
| *Saccharomyces* (control) | 120 | 15 | 0 |
| *Saccharomyces* | 120 | 30 | 51.8 |
| *Saccharomyces* (control) | 120 | 30 | 0 |
| *control experiments ; heat treatment of the suspension in similar dillution value as the premix | | | |

## Claims

**1.** A method for formulating cells or parts of cells, wherin the method comprises the step of (a) mixing a composition containing the cells with at least one matrix material, wherein during the mixing the matrix material absorbs water from the composition comprising the cells.

**2.** A method according to claim 1, further comprising the step of: (b) subjecting the matrix material to a treatment which effects the formation of a continuous phase of the matrix material, whereby step (b) is performed before, during and/or after mixing in step (a).

**3.** A method according to claim 2, wherein the treatment comprises an input of thermal energy whereby the mixture is subjected to a temperature of between 20 °C and 200°C, in particular between 30°C and 120°C, for a time of between 1 min and 48 hrs, and wherein the treatment optionally involves an input of mechanical energy resulting in shear and/or pressure.

**4.** A method according to any one of the preceeding claims, wherein the method further comprises the step of: (c) subjecting the mixture obtained in step (a), or (b) to one or more treatments selected from the group consisting of:
- drying the mixture, (preferably at a temperature of between 30 °C and 120°C, and preferably for a time of between 2 min. and 3 hrs);
- shaping the mixture, (preferably into pellets, granules, fibers, flakes, powder or tablets);
- coating the mixture;
- packaging the mixture;
- storing the mixture;
- transporting the mixture to the location of final use; and,
- dispursing or suspending the mixture.

**5.** A method, according to any of the preceding claims, in which the composition containing the cells is in the form of a suspension of the cells in a liquid medium, and in particular in the form of a suspension of the cells in an aqueous medium.

**5.** A method according to claim 4, in which the density of the cells in the suspension is between 10² and 10¹³ cells per milliliter of liquid/aqueous medium, in particular between 10⁵ and 10¹⁰ cells per milliliter of liquid/aqueous medium

**6.** A method according to any of the preceding claims, in which the matrix material is a polymer, oligomer or a mixture of two or more polymers or oligomers, chosen from:
- one or more biopolymers or oligomers, such as a protein or carbohydrates, or a derivative thereof;
- one or more synthetic polymers;
or any combination thereof.

**7.** A method according to any of the preceding claims, in which the matrix material is a hydrophilic polymer or a mixture of two or more hydrophilic polymers.

**8.** A method according to any of the preceding claims, in which, prior to the mixing in step (a), the matrix material has a water absorption capacity of at least 0.3 g water/ g material.

**9.** A method according to any of the preceding claims, in which the one or more polymer(s) that make up the matrix material are chosen from starch (native or modified), (cyclo)dextrins, inulin, gelatine, casein, proteins from sources like wheat, soy, corn or other grains, hydrocolloids such as carragenans, alginates, xantan gum, gum arabic, guar flour, guar gum, agar, tragacanth, karaya, locust bean gum, fibres, modified celluloses, pectin, cellulose esters, cellulose ethers; or any combination thereof.

**10.** A method according to any of the preceding claims, in which prior to or during step (a) or (b), at least one additive chosen from
- one or more osmotically active agents;
- one or more cryoprotectants;
- one or more hydrophobic components and /or lubricants;
- one or more emulsifiers;
- one or more plasticisers;
- one or more fillers;
or any combination thereof, are incorporated into the mixture.

**11.** A method according to claim 10, in which the additive is mixed with the composition contaning the cells together with, as part of, and/or in admixture with the matrix material.

**12.** A method according to any of the preceding claims, in which the cells are chosen from:
- viruses, viral particles, viroids, and/or (bacterio)phages
- prokaryotic cells;
- eukaryotic cells, including but not limited to yeast cells, fungal cells, cells of algae, animal cells and/or plant cells; and/or
- parts of cells, including but not limited to organelles.

**13.** A method according to any of the preceding claims, in which the cells are living cells and/or viable cells and/or viable viruses.

**14.** A method according to claim 13, in which during step (a) and step (b), the viability of the cells does not decrease by more than 60 %, preferably by no more than 20 %, and in particular by no more than 10% in which the viability of the cells is calculated as: [the total number of viable cells added to, or present in, the mixture] divided by [the total number of cells added to, or present in, the mixture], multiplied by [100%].

**15.** Method according to any of the preceding claims, in which step (b) is carried out by extrusion, drum drying, oven drying, pelletising equipment, compression moulding or centrifugal extrusion.

**16.** Method according to any of the preceding claims, in which step (a) and step (b) are carried out by extrusion.

**17.** A mixture of cells and a matrix material, obtainable and/or obtained via the method of any of claims 1-16.

**18.** A mixture of cells and a matrix material having the general composition:
(1) cell suspension : between 0.1 and 60 wt.%;
(2) matrix material(s) between 30 and 95 wt.%;
(3) further additives between 0 and 50 wt.%;
to a total of 100 wt.% of the cells (1), the matrix materials (2) and the further additives (3), and further **characterized in that** the matrix materials are in a continuous phase encapsulating the cells, whereby at least 50% of the cells are viable.

**19.** A mixture of cells according to claims 18, having a storage life of at least 1month in particular at least 6 to 36 months.

**20.** Use of a mixture according to any one of claims 17 to 19, as a (dairy) starter culture, a shaped article for tissue engineering, an encapsulated *Bifidobacterium* for colon targetting, an oral or mucosal vaccin, an encapsulated cell for hormone secretion, which is optionally used for implantation into a diabetic mammal, an encapsulated probiotic for taste masking or for targetted delivery, an immobilized cell for bioconversion of metabolites or for fermentation, a composition for waste treatment of soil and/or water, a formulation for soil quality improvement, a fertilizer, a formulation for pest control, a composition for diagnostic purposes, an encapsulation for protection against pathogens.
